# EUROPEAN PATENT APPLICATION

(11) **EP 4 740 998 A1**
(43) Date of publication of application: **13.05.2026**
(21) Application number: 25215132.9
(22) Date of filing: 12.11.2025
(51) Int. Cl.: A61N 1/375, A61N 1/05, A61N 1/372

(54) **SYSTEMS FOR IMPLANTING MEDICAL DEVICES**

(30) Priority: 12.11.2024 US 202463719606 P; 10.11.2025 US 202519384951
(71) Applicant: Pacesetter, Inc., Sylmar, CA 91342 (US)
(72) Inventor: GULSETH, Jared, Sylmar, CA 91342 (US); BLUE, Jeremiah, Sylmar, CA 91342 (US); WEBER, Adam, Sylmar, CA 91342 (US); PIKUS, Michael J, Sylmar, CA 91342 (US)
(74) Representative: Barker Brettell Sweden AB

(57) **Abstract**

An attachment device (250) for coupling a biostimulator (200) to a delivery system (100) includes a case (502), a tether dock (504), and a filament (506). The case (502) has a wall (516), a first end (512), and a second end (514) opposite the first end (512). The wall (516) defines a passage (508) and a slot (510). The passage (508) extends between the first end (512) and the second end (514). The slot (510) extends through the wall (516) such that the passage (508) is accessible through the slot (510). The tether dock (504) extends from second end (514) of the case (502). The tether dock (504) defines a groove (524) extending therethrough. The filament (506) has a fixed end (526) and free end (528). The fixed end (526) is attached to the first end (521) of the case (502). The free end (528) includes a bulb (530) that is selectively receivable in the passage (508) of the case (502) through the slot (510).

## Description

### Technical Field

The present disclosure relates to implantable medical devices and related delivery and retrieval systems. More specifically, the present disclosure relates to devices for delivering and retrieving biostimulators, such as leadless cardiac pacemakers, via a catheter-based delivery system.

### Background

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death. Cardiac pacing may be performed by several implantable medical devices including, but not limited to, electrocardiographs ("ECGs"), electroencephalographs ("EEGs"), squid magnetometers, implantable pacemakers, implantable cardioverter-defibrillators ("ICDs"), neurostimulators, electrophysiology ("EP") mapping and radio frequency ("RF") ablation systems, and the like. Hereinafter these cardiac pacing devices may generally be referred to as a biostimulator.

Biostimulators are typically delivered to an intracardial implant site via a delivery system including catheters, sheaths, and/or introducers. In general, a biostimulator may be connected to a catheter in a docked configuration, in which the biostimulator is securely attached to the catheter. In the docked configuration, the catheter may be operated to guide the biostimulator to an implant site. Once the biostimulator is proximate to the implant site, the delivery system may be used to implant the biostimulator into the tissue of the patient.

Once the biostimulator is secured into the tissue, the biostimulator may be moved into a tethered configuration. In the tethered configuration, the delivery system separates from the biostimulator, but remains connected to the biostimulator. For example, two separate and distinct tethers may connect the biostimulator to the catheter in the tethered stated. In the tethered state, an implanting physician may test the biostimulator to make sure that the biostimulator is securely and electrically connected to patient tissue at a desired location. If the physical and/or electrical connection between the biostimulator and the patient tissue is less than optimal, the biostimulator may be re-docked to the catheter so that that the biostimulator may be moved to a better position for implantation. Once the implanting physician is satisfied with the location of the biostimulator within the patient anatomy, the biostimulator is transitioned from the tethered configuration to a release configuration. During the release configuration, the biostimulator disconnects from the catheter.

### Summary

Existing systems and methods, such as those described above, may be susceptible to inadvertent release. Specifically, the tethers may inadvertently release from an attachment feature of the biostimulator during implantation before the implanting physician desires to release the biostimulator from the catheter. Additionally or alternatively, the tethers may become entangled with one another. Entanglement of the tethers may cause inadvertent release or may prevent release of the biostimulator altogether. Accordingly, there is a need for a catheter system that can reliably and predictably couple and decouple to biostimulators. This disclosure relates generally to catheter-based delivery systems and devices for catheter-based delivery systems that tether to biostimulators without inadvertent release or entanglement. Specifically, this disclosure relates to attachment devices that couple a catheter-based delivery system to a biostimulator using a single filament and a single tether.

In an aspect of the present disclosure, an attachment device for coupling a biostimulator to a delivery system includes a case, a tether dock, and a filament. The case has a wall, a first end, and a second end opposite the first end. The wall defines a passage and a slot. The passage extends between the first end and the second end. The slot extends through the wall such that the passage is accessible through the slot. The tether dock extends from the second end of the case. The tether dock defines a groove extending therethrough. The groove is aligned with the slot of the case. The filament has a fixed end and a free end. The fixed end is attached to the first end of the case. The free end includes a bulb that is selectively receivable in the passage of the case through the slot.

In an embodiment, the groove is aligned with the slot of the case.

In an embodiment, the tether dock is configured to receive a tether of the delivery system within the groove to couple the attachment device to the delivery system.

In an embodiment, the filament is be configured to secure the biostimulator to the delivery system when the bulb is received within the passage of the case and to release the biostimulator from the delivery system when the bulb is outside of the passage of the case.

In an embodiment,, the slot of the case is defined to have a first portion and a second portion that is wider than the first portion.

In an embodiment, the bulb may have a width that is less than the width of the second portion of the slot such that the bulb can pass therethrough.

In an embodiment, the filament is made of a metallic material. In another embodiment, the filament may be made of a fibrous, polymeric, non-metallic material.

In an embodiment, the attachment device comprises a biostimulator secured to the attachment tool.

In an embodiment, the attachment device includes a biostimulator and a holding tool. The biostimulator is secured to the attachment device. The attachment device and the biostimulator may be contained within the holding tool. The holding tool may be configured to guide a tether of the delivery system into the tether dock of the attachment device to couple the attachment device and the biostimulator to the delivery system.

In another aspect of the present disclosure, a system includes a catheter, a tether catheter, a tether, a biostimulator, and an attachment device. The catheter has a proximal end and distal end opposite the proximal end. The catheter includes a docking cap disposed on the distal end of the catheter. The tether catheter is disposed within and extends through the catheter. The tether is disposed within and extends through the tether catheter. The biostimulator includes an attachment member. The attachment device includes a case, a tether dock, and a filament. The case has a wall, a first end, and a second opposite the first end. The wall defines a passage and a slot. The passage extends between the first end and the second end. The slot extends through the wall such that the passage is accessible through the slot. The tether dock extends from the second end of the case. The tether dock defines a groove extending therethrough. The groove is preferably aligned with the slot of the case. The tether is preferably received in the groove to secure the attachment device to the tether catheter. The filament has a fixed end and a free end. The fixed end is attached to the first end of the case. The free end includes a bulb that is selectively receivable in the passage of the case through the slot. The filament is attached to the attachment member of the biostimulator to secure the biostimulator to the attachment device. The attachment device has a docked configuration in which the attachment device is disposed within the catheter at a first position such that the biostimulator is engaged with the docking cap, a tethered configuration in which the attachment device is disposed within the attachment device is disposed within the catheter at a second position distal from the first position such that the biostimulator is disengaged with the docking cap and the bulb of the filament remains within the catheter, and a release configuration in which the attachment device is disposed within the catheter at a third position distal from the second position such that the bulb of the filament is outside the catheter and removable from the passage of the case.

In an embodiment, the tether includes a bullet, e.g., a domed or bulbous connector, at a distal end of the tether. The bullet may have a diameter larger than a diameter of the tether.

In an embodiment, the bullet may have a capsule shape.

In an embodiment, the attachment member of the biostimulator defines a hole extending therethrough. The filament may pass through the hole to secure the biostimulator to the attachment device when the attachment device is in the docked configuration or the tethered configuration.

In an embodiment, the filament may be withdrawn from the hole of the attachment member when the attachment device is in the release configuration to decouple the biostimulator from the tether catheter.

In an embodiment, the tether is made of a metallic material. In another embodiment, the filament may be made of a fibrous, polymeric, non-metallic material.

A method of implanting a biostimulator is disclosed. The method includes advancing the biostimulator to an implant site. The biostimulator is attached to a delivery system by an attachment device in a docked configuration thereof. The method also includes fixing the biostimulator to the implant site. The method also includes moving the attachment device to a tether configuration thereof. The method also includes moving the attachment device to a released configuration thereof.

In an example, the method also includes testing the biostimulator while the attachment device is in the tethered configuration and the biostimulator is fixed to the implant site. Testing the biostimulator may include testing physical fixation of the biostimulator to the implant site and that the biostimulator is electrically connected to the implant site.

In an example, the method includes implanting a second biostimulator to another implant site using the delivery system used to implant the biostimulator. Implanting the second biostimulator may include attaching the second biostimulator to a tether of the delivery system with a holding tool.

Further, to the extent consistent, any of the embodiments or aspects described herein may be used in conjunction with any or all of the other embodiments or aspects described herein.

### Brief Description of the Drawings

Various aspects of the present disclosure are described hereinbelow with reference to the drawings, which are not necessarily drawn to scale, which are incorporated in and constitute a part of this specification, wherein:
FIG. 1 is a perspective view of a delivery system in accordance with embodiments of the present disclosure;
FIG. 2 is an exploded view of a distal end of the delivery system of FIG. 1;
FIG. 3 is a side view of an example biostimulator in accordance with embodiments of the present disclosure;
FIG. 4 is front view of the example biostimulator of FIG. 3 implanted within a heart in accordance with embodiments of the present disclosure;
FIG. 5 is a perspective view of an attachment device in accordance with embodiments of the present disclosure;
FIG. 6 is a top view of the attachment device of FIG. 5;
FIG. 7 is a section view of the attachment device of FIG. 5 taken along section line A-A of FIG. 6;
FIG. 8 is a perspective view of the distal end of the delivery system of FIG. 1 including another attachment device in accordance with embodiments of the present disclosure;
FIG. 9 is a perspective view of the distal end of the delivery system of FIG. 1 including another attachment device in accordance with embodiments of the present disclosure;
FIG. 10 is a perspective view of the distal end of the delivery system of FIG. 1 including another attachment device in accordance with embodiments of the present disclosure;
FIG. 11 is a perspective view of the distal end of the delivery system of FIG. 1 including another attachment device in accordance with embodiments of the present disclosure;
FIG. 12 is a perspective view of the distal end of the delivery system of FIG. 1 including another attachment device in accordance with embodiments of the present disclosure;
FIG. 13 is a perspective view of the distal end of the delivery system of FIG. 1 including another attachment device in accordance with embodiments of the present disclosure;
FIG. 14 is a perspective view of a loading system in accordance with embodiments of the present disclosure;
FIG. 15 is a perspective view of the loading system of FIG. 14 with a top cover removed;
FIG. 16 is a flowchart illustrating a method of implanting a biostimulator;
FIG. 17 is side view of a delivery system including the attachment device of FIG. 5 in a docked configuration in accordance with embodiments of the present disclosure;
FIG. 18 is a section view taken along section line B-B of FIG. 17 of the delivery system of FIG. 17 with the attachment device in a tethered configuration; and
FIG. 19 is a section view taken along section line B-B of FIG. 17 of the delivery system of FIG. 17 with the attachment device in a decoupling configuration.

### Detailed Description

The present disclosure will now be described more fully hereinafter with reference to example embodiments thereof with reference to the drawings in which like reference numerals designate identical or corresponding elements in each of the several views. These example embodiments are described so that this disclosure will be thorough and complete, and will fully convey the scope of the disclosure to those skilled in the art. Features from one embodiment or aspect can be combined with features from any other embodiment or aspect in any appropriate combination. For example, any individual or collective features of method aspects or embodiments can be applied to apparatus, product, or component aspects or embodiments and vice versa. The disclosure may be embodied in many different forms and should not be construed as limited to the embodiments set forth herein; rather, these embodiments are provided so that this disclosure will satisfy applicable legal requirements. As used in the specification and the appended claims, the singular forms "a," "an," "the," and the like include plural referents unless the context clearly dictates otherwise. In addition, while reference may be made herein to quantitative measures, values, geometric relationships or the like, unless otherwise stated, any one or more if not all of these may be absolute or approximate to account for acceptable variations that may occur, such as those due to manufacturing or engineering tolerances or the like.

As used herein, the term "clinician" refers to a doctor, a nurse, or any other care provider and may include support personnel. The use of relative terms throughout the description may denote a relative position or direction. For example, "distal" may indicate a first direction. Similarly, "proximal" may indicate a second direction, opposite to the first direction. Such terms are provided to establish relative frames of reference, however, and are not intended to limit the use or orientation of a system to a specific configuration described in the various embodiments below.

Embodiments of the present disclosure provide systems, devices and methods of restricting, restraining, or otherwise limiting movement of attachment devices of a catheter-based delivery system along a delivery, advancing, or tethering path of the delivery system. The attachment devices described herein may couple a biostimulator to a delivery system for delivery to an implantation site within a patient. The biostimulator may be any one of various types of implantable devices such as, but not limited to, an implantable pacemaker, a leadless pacemaker, an implantable cardioverter-defibrillator ("ICD"), defibrillator, cardiac rhythm management ("CRM") device, neurostimulator, or the like. Specifically, the attachment devices may secure the biostimulator such that the biostimulator does not become inadvertently released from the delivery system within a patient.

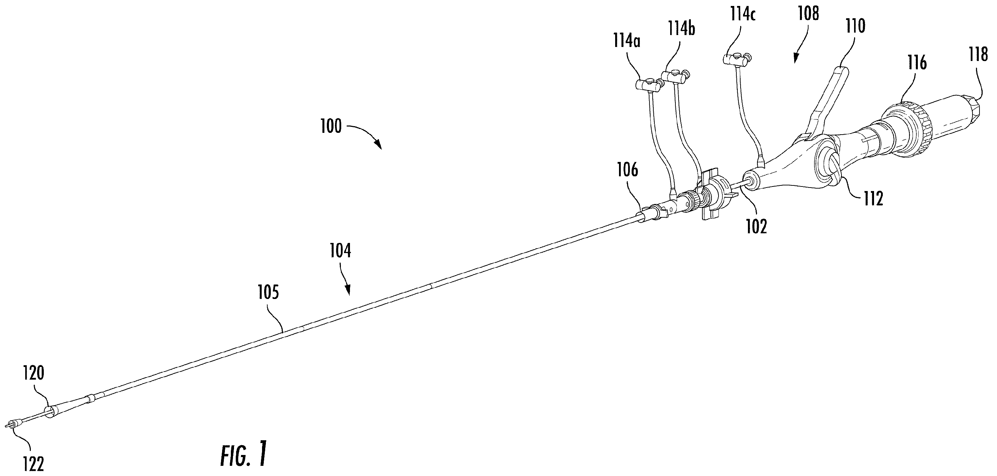

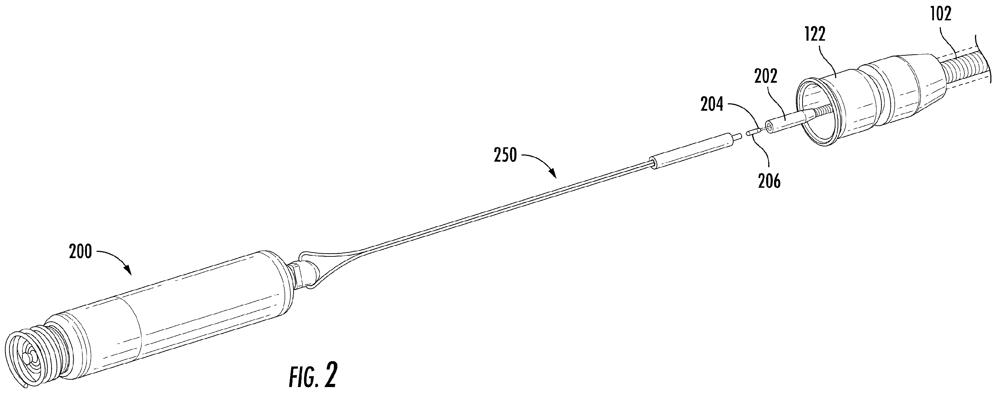

Referring now to FIG. 1 and 2, a delivery system 100 for delivery and/or retrieval of a biostimulator 200, e.g., a leadless pacemaker, into or from a patient is shown. The delivery system 100 may be a catheter-based delivery system. The delivery system 100 can include a deflectable catheter 102, a guide catheter 104, and an introducer sheath 106. The deflectable catheter 102 extends through the guide catheter 104 and includes a distal end and a proximal end. The distal end of the deflectable catheter 102 is selectively connectable to the proximal end of the biostimulator 200 and the proximal end of the deflectable catheter 102 includes a handle 108 by which the user may cause the deflectable catheter 102 to distally-proximally displace within the length of the guide catheter 104 and, further, by which the user may actuate the distal end of the deflectable catheter 102 to selectively connect and disconnect from a proximal end of the biostimulator 200. In embodiments, the delivery system 100 may include a plurality of flush ports 114a, 114b, and 114c. The flush ports 114a, 114b, and 114c may be used to flush saline or other fluids through the introducer sheath 106, the guide catheter 104, and the deflectable catheter 102, respectively.

The guide catheter 104 extends through the introducer sheath 106 and includes a distal end and a proximal end. The distal end of the guide catheter 104 includes a protective sheath 120. A shaft 105 of the guide catheter 104 may also include one or more sections having different durometers such that the reinforcement and corresponding bending resistance of the sections may be modified according to the specific application for which the delivery system 100 is being implemented. The deflectable catheter 102 extends through the guide catheter 104 and terminates in a docking cap 122 that receives a portion of the biostimulator 200 as described below.

The handle 108 may further include additional elements to manipulate and actuate elements of the delivery system 100. In general, the handle 108 may include elements for, without limitation, one or more of deflecting the deflectable catheter 102, rotating the deflectable catheter 102 (and the biostimulator 200, coupled to the deflectable catheter 102), extending and retracting the biostimulator 200 relative to the protective sheath 120, and engaging or disengaging a coupling mechanism, such as the attachment device 250 described below, to a corresponding feature of the biostimulator 200 to couple the biostimulator 200 to the delivery system 100. For example, the handle 108 may include a deflection lever 110 for actuation of the deflectable catheter 102 and a brake 112 for locking the position or otherwise increasing resistance to rotation of the deflection lever 110. The handle 108 may further include a docking shroud 116 that may rotate to apply torsion to the shaft of the deflectable catheter 102, thereby rotating the deflectable catheter 102 and the biostimulator 200 when coupled to the deflectable catheter 102. The docking shroud 116 may also translate along the handle 108 to selectively extend and retract the biostimulator 200 from the protective sheath 120 disposed at a distal end of the shaft 105 of the guide catheter 104. The handle 108 may include a release knob 118 that, when rotated, causes engagement or disengagement of the coupling mechanism with the biostimulator 200.

With particular reference to FIG. 2 a close-up, exploded view of the distal end portion of the delivery system 100 is shown in accordance with embodiments of the present disclosure. The most distal end of the deflectable catheter 102 includes the docking cap 122 and receives the proximal end of the biostimulator 200 for delivery into or withdrawal from a patient. A tether catheter 202 and a tether 204 may extend through the deflectable catheter 102. More particularly, the tether catheter 202 may extend through the deflectable catheter 102 and the tether 204 may extend through the tether catheter 202. The tether catheter 202 and the tether 204 may be extended from or be withdrawn into the deflectable catheter 102 through the docking cap 122. The tether catheter 202 and the tether 204 may be extended or be withdrawn into deflectable catheter 102 together, in concert, or may be extended or withdrawn independently of each other. The tether catheter 202 and the tether 204 cooperate to secure the deflectable catheter 102 with an attachment device 250 that selectively couples the biostimulator 200 to the deflectable catheter 102.

Specifically, the distal end of the tether 204 includes a bullet 206 that is received by the attachment device 250 to secure the attachment device 250 to the deflectable catheter 102. The bullet 206 can have a bulbous, rounded, or enlarged shape or size, relative to a tether. For example, as shown, the bullet can have a capsule shape. In embodiments, the bullet 206 may be, but is not limited to, features on the tether 204 that protrude radially from the tether 204, such as bumps, spheres, cylinders, rectangles, or other similar shapes extending outwards from the tether 204. In some embodiments, the bullet 206 may be expandable, such as a balloon or an expandable mechanical structure. Generally, the bullet 206 has a cross-sectional diameter larger than the cross-sectional diameter of the tether 204. In embodiments, the bullet 206 and the tether 204 may be monolithically formed. The tether 204 may include wires, shafts, tubes, cords, ropes, strings, or other similar structures that can extend through the tether catheter 202. In embodiments, the tether 204 may be made of a shape memory material, such as nitinol. In some embodiments, the tether 204 may be stainless steel wires or braids. Specifically, the tether 204 has a column strength that allows the tether 204 to be advanced through the tether catheter 202 without buckling. In some embodiments, the material of the tether 204 may be chosen for the fatigue life of material. For example, during implantation of the biostimulator 200, the tether 204 may be repeatedly deformed, e.g., bent, crimped, twisted, etc., as the delivery system 100 navigates a torturous path to the implant site. The tether 204 being made of a material capable of withstanding high-cycle deformation may reduce the likelihood of breakage during implantation procedures.

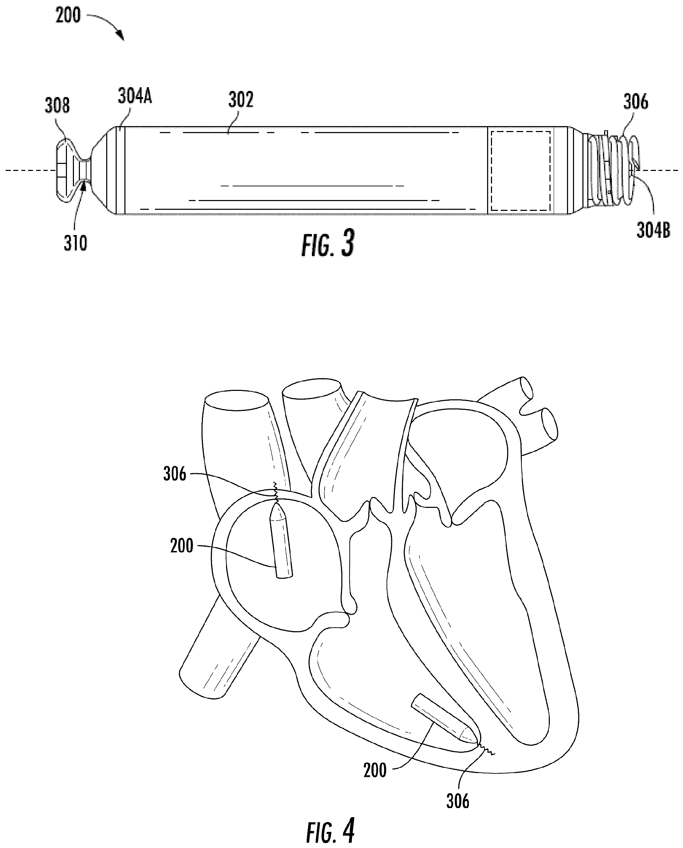

Referring to FIGS. 3 and 4, an example biostimulator 200 is shown. The biostimulator 200 may include a housing 302 and one or more electrodes 304. For example, the electrode(s) may include a shock electrode and/or a sensing electrode. The electrode(s) can include a proximal electrode 304A, which may provide an anode, and a distal electrode 304B, which may provide a cathode. The electrode(s) may deliver pacing pulses to muscle of a cardiac chamber. In some embodiments, one or more of the electrodes 304 are configured to sense electrical activity from the muscle. In particular embodiments, each electrode 304 may deliver pacing pulses to the muscles of the cardiac chamber and sense electrical activity from the muscle. The housing 302 can contain electronics such as a communications coil or antenna, a battery, or a pulse generator. The biostimulator 200 may communicate bidirectionally with at least one other device within or outside the body of the patient. In some embodiments, the biostimulator 200 may be implanted intracardially within a chamber of the heart, as shown in FIG 4. In such embodiments, the biostimulator 200 may include one or more fixation elements 306. For example, the fixation element 306 may be a helical member to allow the biostimulator 200 to be screwed into the target tissue. The biostimulator 200 may have an attachment member 308 for securing the biostimulator 200 to the attachment device 250. The attachment member 308 may be a button, as shown, that defines a hole 310, internal to a neck of the member, to receive a portion of the attachment device 250 therethrough. The portion secures the biostimulator 200 to the attachment device 250 and, thus, to the deflectable catheter 102.

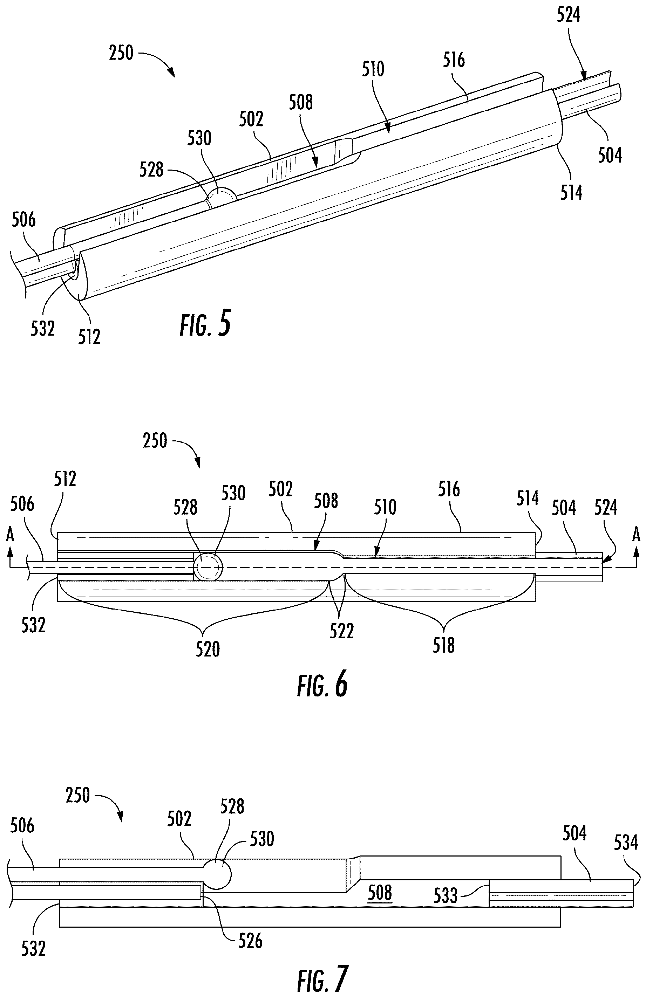

Referring to FIGS. 5-7, an attachment device 250 in accordance with embodiments of the present disclosure is shown. The attachment device 250 is configured to selectively couple the biostimulator 200 to the deflectable catheter 102 and, more particularly to the docking cap 122, for delivery to an implant site. The attachment device 250 includes a case 502, a tether dock 504, and a filament 506. The tether dock 504 receives the tether 204 to secure the attachment device 250 to the tether catheter 202. The filament 506 secures with the attachment member 308 of the biostimulator 200 to secure the biostimulator 200 to the attachment device 250 and, thus, couple the biostimulator 200 to the deflectable catheter 102.

The case 502 is generally tubular and defines a passage 508 and a slot 510. The passage 508 extends through the case 502 from a first end or case distal end 512 to a second end or case proximal end 514. The slot 510 extends through a wall 516 of the case 502 such that the passage 508 is accessible through the slot 510. The slot 510 may be defined to have narrow portion 518, also referred to as first portion herein and a wide portion 520, also referred to as second portion herein. The narrow portion 518 may be disposed nearer the case proximal end 514 and the wide portion 520 may be disposed nearer the case distal end 512. As such, the profile of the slot 510 may be considered generally bottle shaped. The slot 510 may include a transition portion 522 between the narrow portion 518 and the wide portion 520. The transition portion 522 may conform to the shape of the bullet 206, or a portion of the bullet 206. For example, as shown, the transition portion 522 has a radius that conforms to the capsule shape of the bullet 206. The narrow portion 518 of the slot 510 may have a width greater than a diameter of the tether 204 but less than a diameter of the bullet 206.

The tether dock 504 receives the tether 204 to couple the attachment device 250 to the deflectable catheter 102 and, more specifically, to the tether catheter 202. The tether dock 504 extends from the case proximal end 514. The tether dock 504 is also generally tubular and defines a groove 524. The groove 524 extends the longitudinal length of the tether dock 504 from a tether dock distal end 533 to a tether dock proximal end 534. The tether dock 504 extends from the case proximal end 514 with the groove 524 aligned with the slot 510 of the case 502. The groove 524 may have a width substantially equal to the width of the narrow portion 518 of the slot 510. A length of the tether dock 504 may be disposed within the passage 508 of the case 502, as shown. In some embodiments, the tether dock 504 and the case 502 are monolithically formed. In such an embodiment, the tether dock 504 may extend directly from the case proximal end 514 of the case 502. The tether dock 504 may abut the tether catheter 204 when secured to the tether catheter 204. In embodiments, the tether dock 504 may be received within the tether catheter 204 when secured to the tether catheter 204.

The filament 506 secures the biostimulator 200 to attachment device 250 and, thus, to the deflectable catheter 102. The filament 506 has a fixed end 526 and a free end 528. The free end 528 includes a bulb 530. The fixed end 526, or a segment of the filament 506 adjacent to the fixed end 526, is fixed to the case distal end 512. The fixed end 526 extends to the free end 528 and terminates in the bulb 530. The bulb 530 protrudes radially from the filament 506, such as bumps, spheres, cylinders, rectangles, or other similar shapes extending outwards from the filament 506. In some embodiments, the bulb 530 may be expandable, such as a balloon or an expandable mechanical structure. Generally, the bulb 530 has a cross sectional diameter larger than the cross-sectional diameter of the filament 506. The filament 506 may be formed of a flexible or semi-flexible material. The filament 506 may be a metallic material or a non-metallic material. The filament 506 may include wires, shafts, tubes, cords, ropes, strings, or other similar structures. In embodiments, the filament 506 may be made of a shape memory material, such as nitinol. In some embodiments, the filament 506 may be metallic, e.g., stainless steel, wires, coils, or braids. In certain embodiments, the filament 506 may be a fibrous, polymeric, non-metallic material. For example, the tether 204 may be made of ultra-high molecular weight polyethylene (UHMWPE), polyester (PE), Kevlar^{®}, or Vectran^{®}. In some embodiments, the material of the filament 506 may be chosen for the fatigue life of material. For example, during implantation of the biostimulator 200, the filament 506 may be repeatedly deformed, e.g., bent, crimped, twisted, etc. The filament 506 being made of a material capable of withstanding high-cycle deformation may reduce the likelihood of breakage during implantation procedures. The filament 506 may be disposed within a beating heart for a duration in a range of 10 minutes to 30 minutes, e.g., 20 minutes. More particularly, the filament 506 may be configured to withstand a number of loading cycles, e.g., heart beats, for the duration the filament 506 is disposed within the heart. In embodiments, the filament 506 may be configured to withstand a number of cycles in the range of 600 cycles to 3,000 cycles. For example, where the filament 506 is disposed within a heart beating at 60 beats per minute for twenty minutes, the filament 506 may be exposed to 1,200 loading cycle. The free end 528 of the filament 506 may be doubled back so that the bulb 530 can be received in the passage 508 through the slot 510 and a bight is formed between the fixed end 526 and the free end 528.

The fixed end 526 of the filament 506 may include a plug 532. The plug 532 may be received in the passage 508 of the case 502 and fix the filament 506 to case 502. For example, the plug 532 may be crimped to fix the filament 506 to the plug 532 and the plug 532 may be welded to the case 502. The plug 532 may be disposed entirely within the passage 508 of the case 502, as shown. In embodiments, the plug 532 may extend from the case distal end 512, similarly to the tether dock 504.

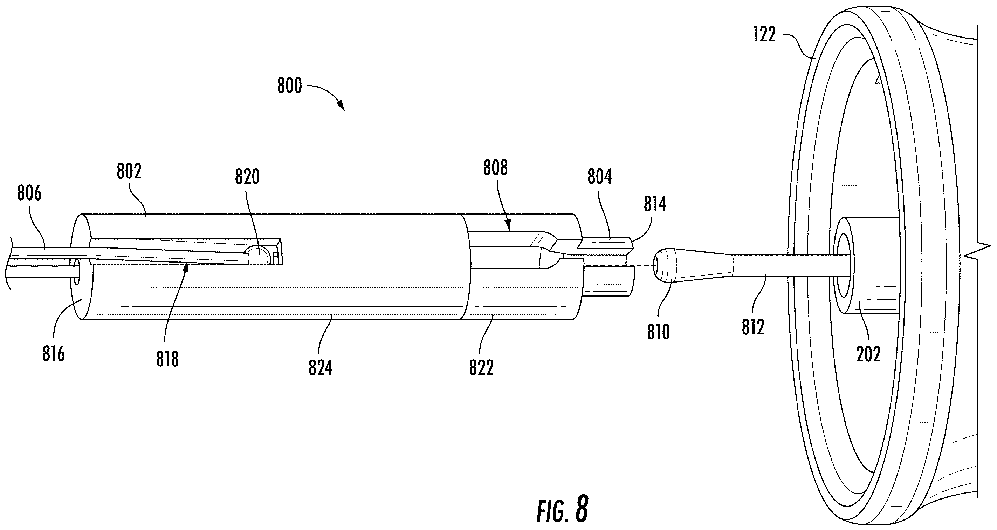

Referring to FIG. 8, another example attachment device 800 in accordance with embodiments of the present disclosure is described. For reasons of brevity only the differences between the attachment device 800 and the attachment device 250 will be described herein.

The attachment device 800 includes a case 802, a tether dock 804, and a filament 806. The case 802 defines a receiver 808 to receive a bullet 810 of the tether 812. The receiver 808 extends partially through the case 802 from a case proximal end 814 towards a case distal end 816. The bullet 810 may have a tapered profile, e.g., a generally teardrop shaped profile, as shown. The case 802 may define a recess 818 to receive a bulb 820 of the filament 806. The recess 818 extends partially through the case 802 from the case distal end 816 towards the case proximal end 814. The recess 818 may be sloped such that the recess 818 is deeper nearer the case proximal end 814 than nearer the case distal end 816. The slope of the recess 818 may provide space to accommodate the bulb 820. In embodiments, the case 802 may have a monolithic construction. In some embodiments, the case 802 may include a tether portion 822 and a filament portion 824 that are attached to each other. For example, the tether portion 822 and the filament portion 824 may be threadably attached to each other.

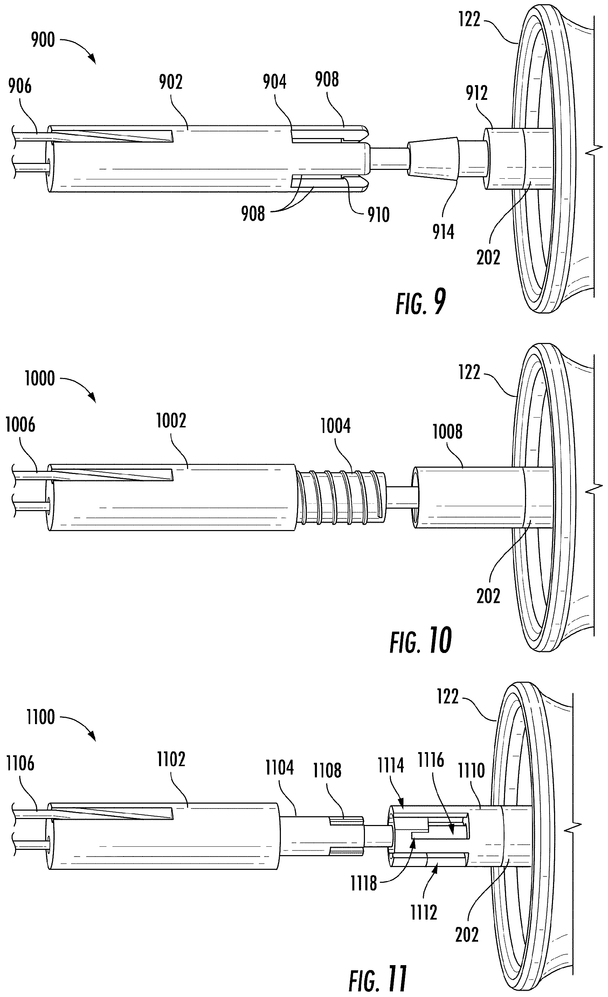

Referring to FIG. 9, another example attachment device 900 in accordance with embodiments of the present disclosure is described. For reasons of brevity only the differences between the attachment device 900 and the attachment devices 250 and 800 will be described herein.

The attachment device 900 includes a case 902, a cap dock 904, and a filament 906. The cap dock 904 includes a plurality of fingers 908. Each finger 908 includes a shelf 910 extending toward the central longitudinal axis of the case 902. The fingers 908 may be resilient members configured to deflect from and return to a neutral position. More particularly, a retention cap 912 is disposed on the end of the tether catheter 202 and is configured to be received between the fingers 908 and retain the attachment device 900 to the tether catheter 202. The retention cap 912 may have a frustoconical profile that urges the fingers 908 apart, away from the neutral position, when pushed between the fingers 908. The retention cap 912 forms a ledge 914 that cooperates the shelves 910 to lock the case 902 to the retention cap 912. When the shelves 910 and of the fingers 908 engage the ledge 914, the fingers 908 may return the neutral position. With the ledge 914 engaged with the shelves 910 the attachment device 900 is secured to the tether catheter 202.

Referring to FIG. 10, another example attachment device 1000 in accordance with embodiments of the present disclosure is described. For reasons of brevity only the differences between the attachment device 1000 and attachment devices 250 and 800 will be described herein.

The attachment device 1000 includes a case 1002, a cap dock 1004, and filament 1006. The cap dock 1004 may be threaded to threadably couple the attachment device 1000 to a retention cap 1008 disposed on the end of the tether catheter 202. The retention cap 1008 may have cooperating threads to couple with the cap dock 1004. The cap dock 1004 may have male threads and the retention cap 1008 may have female threads to receive the cap dock 1004. In some embodiments, the cap dock 1004 may have female threads and the retention cap 1008 may have male threads to be received by the retention cap 1008. The threads of the cap dock 1004 and the retention cap 1008 may be right or left handed threads. In embodiments, when the attachment device 1000 is secured with the retention cap 1008, the interface between the retention cap 1008 and the case 1002 may be substantially smooth. Specifically, the outer diameter of the case 1002 and the outer diameter of the retention cap 1008 may be substantially equal such that, when the cap dock 1004 is received in the retention cap 1008, the outer surface of the case 1002 and the outer surface of the retention cap 1008 are substantially flush.

Referring to FIG. 11, another example attachment device 1100 in accordance with embodiments of the present disclosure is described. For reasons of brevity only the differences between the attachment device 1100 and the attachment devices 250 and 800 will be described herein.

The attachment device 1100 includes a case 1102, a cap dock 1104, and filament 1106. The cap dock 1104 includes a plurality of tabs 1108 extending outwardly, away from the central longitudinal axis of the case 1102. A retention cap 1110 may be disposed on the distal end of the tether catheter 202. The retention cap 1110 defines a plurality of notches 1112 to receive the tabs 1108 of the cap dock 1104. The notches 1112 may be generally J-shaped such that each notch 1112 has a gate 1114, a bridge 1116, and a pocket 1118. The gates 1114 and the pockets 1118 of each notch 1112 may be parallel to each other. The bridge 1116 extends between and connects the gates 1114 and the pocket 1118. To secure the attachment device 1100 to the retention cap 1110, the cap dock 1104 is inserted into the retention cap 1110 with the tabs 1108 aligned with a respective one the notches 1112. Specifically, the tabs 1108 are aligned with and inserted into the gates 1114 of the respective notches 1112. Once the tabs 1107 are fully inserted into the gate 1114, the case 1102 may be rotated to move the tabs 1108 through the bridge 1116 and into the pocket 1118. For example, the gate 1114 and the pocket 1118 may extend longitudinally, and the bridge 1116 may extend circumferentially between the gate 1114 and the pocket 1118. The pockets 1118 selectively retain the tabs 1108 and, thus, secures the attachment device 1100 to the retention cap 1110. In embodiments, the case 1102 or the retention cap 1110 may include a spring to urge the case 1102 and the retention cap 1110 apart. The urging of the spring may resist unintended decoupling of the attachment device 1100 and the retention cap 1110. Specifically, the spring may urge the tabs 1108 into a respective pocket 1118 to resist decoupling of the attachment device 1100.

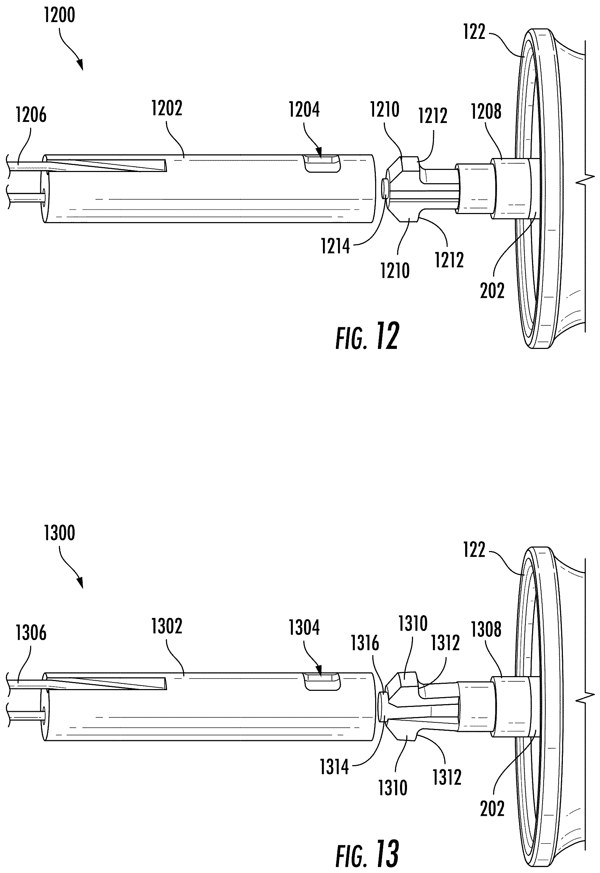

Referring to FIG. 12, another example attachment device 1200 in accordance with embodiments of the present disclosure is described. For reasons of brevity only the differences between the attachment device 1100 and the attachment devices 250 and 800 will be described herein.

The attachment device 1200 includes a case 1202, a cap dock 1204, and a filament 1206. The cap dock 1204 is a pair of ports defined in the case 1202. A retention cap 1208 may be disposed on the distal end of the tether catheter 202. The retention cap 1208 is configured to be received in the cap dock 1204 to secure the attachment device 1200 to the tether catheter 202. The retention cap 1208 includes a pair of fingers 1210. Each finger 1210 includes a shelf 1212 extending outwardly from the retention cap 1208. The fingers 1210 may be resilient members configured to deflect from and return to a neutral position. More particularly, a lock rod 1214 may be disposed between the fingers 1210 to urge the fingers 1210 apart, away from the neutral position. In embodiments, the lock rod 1214 may be a conventional guide wire for catheter-based systems. To secure the attachment device 1200 to the tether catheter 202, the retention cap 1208 may be inserted into the case 1202 with the shelf 1212 of each finger 1210 aligned with a respective port of the cap dock 1204. The lock rod 1214 may be advanced, e.g., moved distally, to be positioned between the fingers 1210 to urge the fingers 1210 apart and each shelf 1212 into a respective port to prevent decoupling of the attachment device 1200 from the retention cap 1208. When the lock rod 1214 is retracted, e.g., moved proximally, from between the pair of fingers 1210, the fingers 1210 may return to a neutral position such that the shelves 1212 are withdrawn from the ports of the cap dock 1204 and, thus, allow for decoupling of the attachment device 1200 from the retention cap 1208. In embodiments, features of the handle 108 may allow for selective locking of the position of the lock rod 1214 to prevent unintended decoupling of the attachment device 1200.

Referring to FIG. 13, another example attachment device 1300 in accordance with embodiments of the present disclosure is described. For reasons of brevity only the differences between the attachment device 1300 and the attachment devices 250 and 800 will be described herein.

The attachment device 1300 includes a case 1302, a cap dock 1304, and a filament 1306. The cap dock 1304 is a pair of ports defined in the case 1302. A retention cap 1308 may be disposed on the distal end of the tether catheter 202. The retention cap 1308 may be configured to be received in the cap dock 1304. The retention cap 1308 may include a pair of fingers 1310. Each finger 1310 includes a shelf 1312 extending outwardly from the retention cap 1308. The fingers 1310 may be resilient members configured to deflect from and return to a neutral position. More particularly, a lock rod 1314 may be disposed between the fingers 1310 to urge the fingers 1310 apart, away from the neutral position. In embodiments, the lock rod 1314 may be a conventional guide wire for catheter-based systems. In some embodiments, the lock rod 1314 may include a wedge 1316 having a tapered profile to urge the fingers 1310 apart. To secure the attachment device 1300 to the tether catheter 202, the retention cap 1308 may be inserted into the case 1302 with the shelf 1312 of each finger 1310 aligned with a respective port of the cap dock 1304. The lock rod 1314 may then be withdrawn, e.g., moved proximally, to be positioned between the fingers 1310 to urge the fingers 1310 apart and each shelf 1312 into a respective port of the cap dock 1304 to prevent decoupling of the attachment device 1300 from the retention cap 1308. When the lock rod 1314 is advanced, e.g., moved distally, the wedge 1316 may move from between the pair of fingers 1310 and the pair of fingers 1310 may return to a neutral position such that the shelves 1312 are withdrawn from the ports of the cap dock 1304 and allow for decoupling of the attachment device 1300 from the retention cap 1308. In embodiments, features of the handle 108 may allow for selective locking of the position of the lock rod 1314 to prevent unintended decoupling of the attachment device 1300.

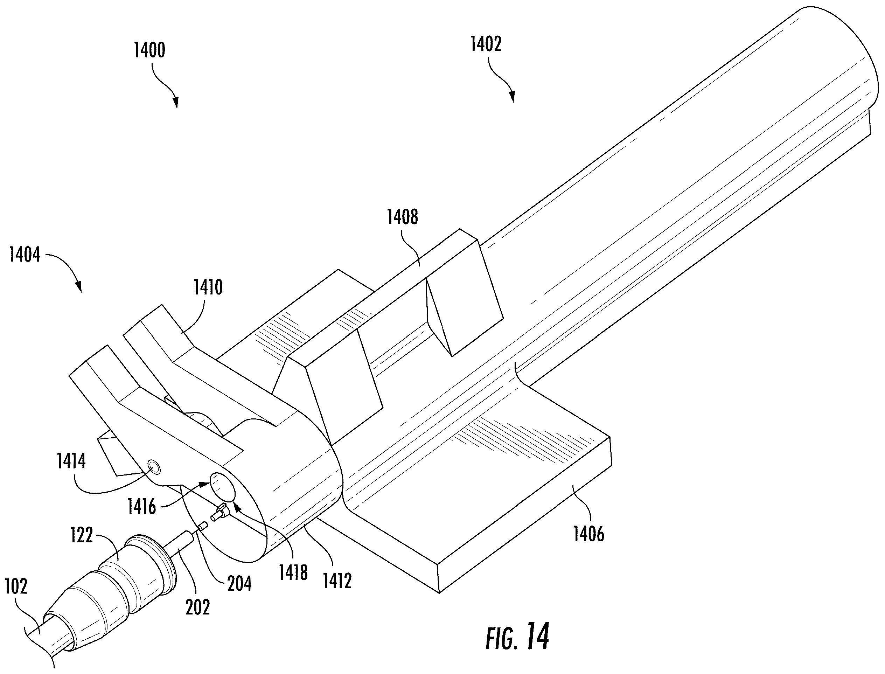

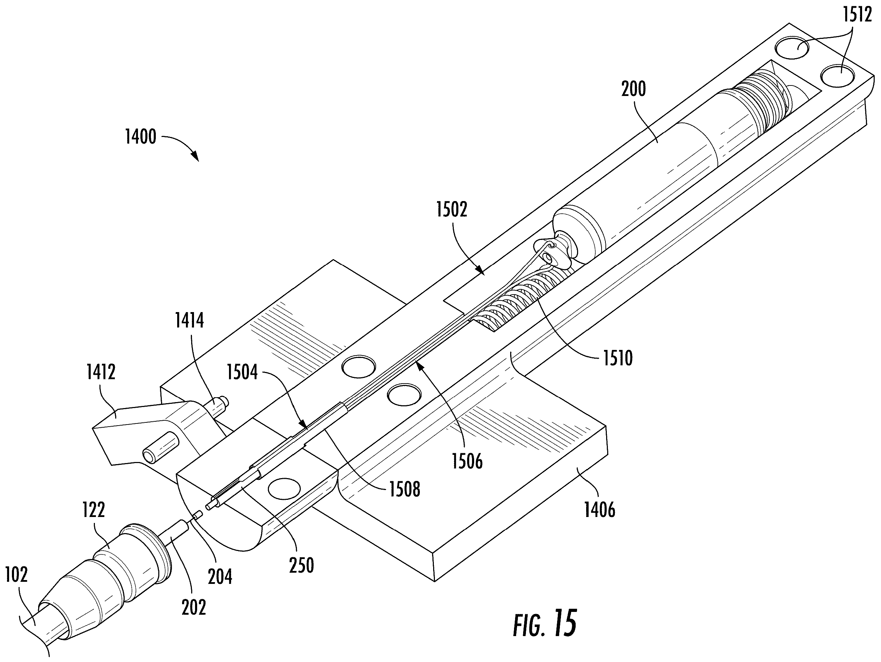

Referring to FIGS. 14 and 15, a loading system 1400 for coupling the attachment device 250 and the biostimulator 200 to the deflectable catheter 102 in accordance with embodiments of the present disclosure is shown. The loading system 1400 contains the biostimulator 200 and the attachment device 250 to allow for coupling of the attachment device 250 to the tether 204 by a clinician. Specifically, the loading system 1400 guides the tether 204 into the tether dock 504 to secure the attachment device 250 to the tether 204 and, thus, the biostimulator 200 to the delivery system 100.

The loading system 1400 includes a holding tool 1402 and an alignment tool 1404. The holding tool 1402 has a receptacle 1406 and a cover 1408. The receptacle 1406 defines a cavity 1502, a socket 1504, and a filament channel 1506. The cavity 1502 receives and contains the biostimulator 200. The socket 1504 may receive a portion of the attachment device 250. In some embodiments, the socket 1504 receives a collet 1508 that may receive a portion of the attachment device 250. The collet 1508 may deflect when cover 1408 is mated with receptacle 1406 to clamp the attachment device 250. The collet 1508 may be a modified hypodermic tube, e.g., with a cutout to allow for more deflection. The cavity 1502 and the socket 1504 are connected by a filament channel 1506. The filament channel 1506 allows the filament 506 of the attachment device 250 to extend between the cavity 1502 and the socket 1504. The receptacle 1406 may include a tensioner 1510 within the cavity 1502. The tensioner 1510 maintains tension on the filament 506 so that the bulb 530 does not inadvertently dislodge from the case 502. In embodiments, the tensioner 1510 may be a spring, e.g., a coil spring, that urges the biostimulator 200 away from the socket 1504. In some embodiments, the collet 1508 may cover the slot 510 to retain the bulb 530 within the passage 508 of the case 502. The receptacle 1406 and the cover 1408 may be mated together. For example, the receptacle 1406 and the cover 1408 may include magnets 1512 to secure the receptacle 1406 and the cover 1408 together.

The alignment tool 1404 includes a top aligner 1410 and a bottom aligner 1412. The attachment device 250 is received between the top aligner 1410 and the bottom aligner 1412 to hold the slot 510 of the case 502 at a fixed radial orientation. The top aligner 1410 and the bottom aligner 1412 are pivotally coupled to each other about a pin 1414. The top aligner 1410 and the bottom aligner 1412 may pivot about the pin 1414 between a closed state and an open state. In the closed state, the top aligner 1410 and the bottom aligner 1412 are engaged with each other and hold the attachment device 250 at a fixed radial orientation. The alignment tool 1404 may be biased toward the closed state. For example, the top aligner 1410 and the bottom aligner 1412 may be urged together by a spring, e.g., a torsional spring. The alignment tool 1404 may receive a portion of the collet 1508 between the top aligner 1410 and the bottom aligner 1412 and may deflect a portion of the collet 1508 when in the closed state to clamp the attachment device 250 and fix the alignment between the slot 510 and the tether gate 1418. In the open state the bottom aligner 1412 are spaced apart such that the case 502 of the attachment device 250 may be removed from or inserted into the loading system 1400. The top aligner 1410 defines a funnel 1416 having a tether gate 1418. When the alignment tool 1404 is in the closed state with the attachment device 250 between the top aligner 1410 and the bottom aligner 1412 the slot 510 of the case 502 may be aligned with the tether gate 1418. Accordingly, the tether 204 may be guided into the tether dock 504 by the loading system 1400.

In embodiments, the loading system 1400 may be a kit including the attachment device 250 and the biostimulator 200. The kit may be delivered to the clinician prepared for coupling to the tether 204. Specifically, the kit contains the biostimulator 200 within the cavity 1502 of the holding tool 1402 and the attachment device 250 held between the top aligner 1410 and the bottom aligner 1412 of the aligner tool 1404 with the filament 506 extending through the filament channel 1506 connecting the biostimulator 200 and the attachment device 250. In particular, the filament 506 is passed through the hole 310 of the attachment member 308 of the biostimulator 200 and is doubled back through the filament channel 1506 with the bulb 530 of the filament 506 disposed in the passage 508 of the case 502. As such, the attachment member 308 of the biostimulator 200 is secured to the filament 506 along the bight formed between the fixed end 526 and the free end 528 of the filament 506. The attachment device 250 is held between the top aligner 1410 and the bottom aligner 1412 with the slot 510 aligned with the tether gate 1418. In embodiments, the kit may be sterile and aseptically sealed.

For a clinician to secure the tether 204 to the attachment device 250, the tether 204, and more specifically the bullet 206, may be inserted into the funnel 1416 to guide the bullet 206 into the passage 508 of the case 502 and the tether 204 into the groove 524 of the tether dock 504. The tether 204 may be retracted into the tether catheter 202 and the tether catheter 202 may be retracted into the deflectable catheter 102 to withdraw the attachment device 250, or a portion of the attachment device 250, into the deflectable catheter 102. In embodiments, the attachment device 250 may be withdrawn into the deflectable catheter 102 directly from the loading system 1400 with the alignment tool 1404 in the closed state. In some embodiments, the alignment tool 1404 may be removed from the attachment device 250 before the attachment device 250 is withdrawn into the deflectable catheter 102. With the attachment device 250 withdrawn into the deflectable catheter 102 the biostimulator 200 may be removed from the holding tool 1402. The biostimulator 200 may be removed by separating the cover 1408 from the receptacle 1406. With the cover 1408 removed, the tether catheter 202 and the tether 204 may be withdrawn further into the deflectable catheter 102 to engage the biostimulator 200 with the docking cap 122.

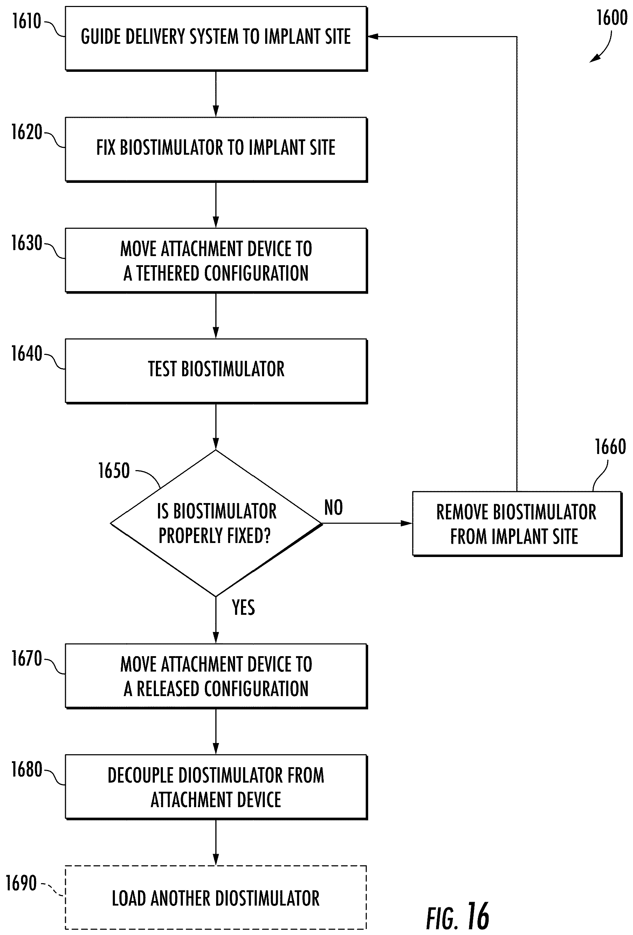

Referring now to FIG. 16, a flowchart illustrating a method 1600 of implanting a biostimulator 200 at an implant site is shown. The method is described with reference to the delivery system 100 of FIGS. 1-15.

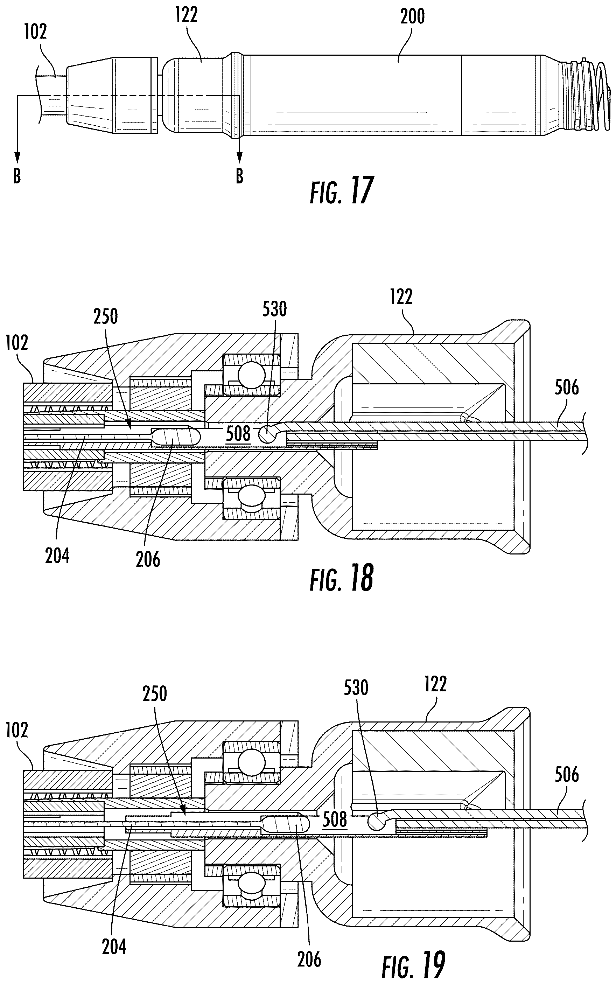

The delivery system 100 including the biostimulator 200 is guided to an implant site, e.g., within the right ventricle of a patient (Step 1610). The deflectable catheter 102 may be extended from the guide catheter 104 with the attachment device 250 in a docked configuration, as shown in FIG. 17. In the docked configuration, the attachment device 250 is withdrawn into the deflectable catheter 102 such that the biostimulator 200 is engaged with the docking cap 122. Once at the implant site, the biostimulator 200 is fixed to the implant site (Step 1620). For example, the biostimulator 200 may be fixed to a wall of a chamber of the heart, as shown in FIG. 4. Fixing the biostimulator 200 may include rotating the biostimulator 200 so that the fixation element 306 of the biostimulator 200 screws into the tissue of the heart. The deflectable catheter 102 may be moved away, e.g., proximally, from the implant site and the attachment device 250 may be advanced, e.g., distally, within deflectable catheter 102 to transition the attachment device 250 to a tethered configuration, as shown in FIG. 18 (Step 1630). The attachment device 250 may be advanced by moving the tether catheter 202 and the tether 204 distally. In the tethered configuration the biostimulator 200 remains attached to the attachment device 250 by the filament 506. More particularly, when the attachment device 250 is in the tethered configuration, the attachment device 250 is advanced distally with respect to the deflectable catheter 102 such that the bulb 530 of the filament 506 remains within the passage 508 of the case 502. The bulb 530 may be retained within the passage 508 by an interior wall of the deflectable catheter 102 or by the docking cap 122.

When the biostimulator 200 is fixed to the implant site and the attachment device 250 is in the tethered configuration, the biostimulator 200 is tested to determine whether the biostimulator 200 is properly physically and electrically connected to the implant site (Step 1640). Based on the test at Step 1640, whether the biostimulator 200 is properly affixed to the implant site is determined (Step 1650). The attachment device 250 being in the tethered configuration during testing may allow the biostimulator 200 to move within the patient without damaging the filament 506. For example, the biostimulator 200 may move as a result of the heart beating or blood flowing through the heart passed the biostimulator 200. If the biostimulator 200 is not properly affixed to the implant sight, then the biostimulator 200 may be removed from the implant site and retracted back into docking cap 122 such that the attachment device 250 is returned to the docked configuration (Step 1660). Once the biostimulator 200 is returned to the docking cap 122, Steps 1610-1650 may be repeated until the biostimulator 200 is properly fixed at the implant site. If the biostimulator 200 is determined to be properly affixed to the implant site during Step 1650 then the attachment device 250 may be moved to a release configuration (Step 1670). With the attachment device 250 in the release configuration, the biostimulator 200 may be unsecured from the attachment device 250 (Step 1680). The attachment device 250 and the biostimulator 200 may be moved to the release configuration by withdrawal of the deflectable catheter 102 away from the implanted biostimulator 200 and/or by advancing the tether catheter 202 and the attachment device 250, as shown in FIG. 19, to position the bulb 530 of the filament 506 outside of the deflectable catheter 102. More particularly, in the release configuration the attachment device 250 is extended at least partially out of the deflectable catheter 102 such that the bulb 530 of the filament 506 may be released from the passage 508 of the case 502 through the slot 510. The tether catheter 202 and the tether 204 may then be withdrawn into the deflectable catheter 102 to pull the filament 506 free from the attachment member 308 of the biostimulator 200. Specifically, moving the tether catheter 202 and the tether 204 proximally into the deflectable catheter 102, away from the implanted biostimulator 200, while the attachment device 250 is in the release configuration the filament 506 is pulled through and free of the hole 310 of the attachment member 308 of the biostimulator 200. As such, the biostimulator 200 may be left implanted in the patient.

In some cases, more than one biostimulator 200 may be implanted by loading another biostimulator 200 into the delivery system 100 and repeating the method 1600 (Step 1690). To load another biostimulator 200, e.g., a second biostimulator 200, into the delivery system 100, the deflectable catheter 102 may be withdrawn from the patient. In embodiments, the guide catheter 104 may remain in the patient while the delivery system 100 is reloaded with the second biostimulator 200. Leaving the guide catheter 104 within the patient may allow for faster implantation of more than one biostimulator 200. Once withdrawn, the attachment device 250, having implanted the biostimulator 200, may be unsecured from the tether 204. Another attachment device 250, e.g., a second attachment device 250, may be secured to the tether 204 with the second biostimulator 200 for implantation. The second attachment device 250 may be attached with the aid of the loading system 1400. As described above, the loading system 1400 may be a kit delivered to the clinician including the second biostimulator 200 and the second attachment device 250 ready to be secured to the tether 204. With the second attachment device 250 and the second biostimulator 200 coupled to the deflectable catheter 102 in the docked configuration, the second biostimulator 200 may be delivered to a second implant sight through the guide catheter 104 that remains in the patient.

Although the method steps are described in a specific order, it should be understood that other steps may be performed in between described steps, described steps may be adjusted so that they occur at slightly different times, or the described steps may occur in any order unless otherwise specified.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

## Claims

1. An attachment device (250) for coupling a biostimulator (200) to a delivery system (100), the attachment device (250) comprising:
a case (502) having a wall (516), a first end (512), and a second end (514) opposite the first end (512), the wall (516) defining a passage (508) and a slot (510), the passage (508) extending between the first end (512) and the second end (514), the slot (510) extending through the wall (516) such that the passage (508) is accessible through the slot (510);
a tether dock (504) extending from the second end (514) of the case (502), the tether dock (504) defining a groove (524) extending therethrough; and
a filament (506) having a fixed end (526) and a free end (528), the fixed end (526) attached to the first end (512) of the case (502), the free end (528) including a bulb (530) that is selectively receivable in the passage (508) of the case (502) through the slot (510).

2. The attachment device of claim 1, wherein the groove (524) is aligned with the slot (510) of the case (502).

3. The attachment device according to claim 1 or 2, wherein the tether dock (504) is configured to receive a tether (204) of the delivery system (100) within the groove (524) to couple the attachment device (250) to the delivery system (100).

4. The attachment device according to any one of claims 1 to 3, wherein the filament (506) is configured to secure the biostimulator (200) to the delivery system (100) when the bulb (530) is received within the passage (508) of the case (502), and wherein the filament (506) is configured to release the biostimulator (200) from the delivery system (100) when the bulb (530) is outside of the passage (508) of the case (502).

5. The attachment device according to any one of claims 1 to 4, wherein the slot (510) of the case (502) is defined to have a first portion (518) and a second portion (520) that is wider than the first portion.

6. The attachment device according to claim 5, wherein the bulb (530) has a width less than the width of the second portion (520) of the slot (510) such that the bulb (530) can pass therethrough.

7. The attachment device according to any one of claims 1 to 6, wherein the filament (506) is made of a metallic material.

8. The attachment device according to any one of claims 1 to 6, wherein the filament (506) is made of a fibrous, polymeric, non-metallic material.

9. The attachment device according to any one of claims 1 to 8, further comprising a biostimulator (200) secured to the attachment device (250).

10. The attachment device according to claim 9, further comprising a holding tool (1402), the attachment device (250) and the biostimulator (200) contained within the holding tool (1402), the holding tool (1402) configured to guide a tether (204) of the delivery system (100) into the tether dock (504) of the attachment device (250) to couple the attachment device (250) and the biostimulator (200) to the delivery system (100).

11. A system comprising:
a catheter (102) having a proximal end and a distal end opposite the proximal end, the catheter (102) including a docking cap (122) disposed on the distal end of the catheter (102);
a tether catheter (202) disposed within and extending through the catheter (102);
a tether (204) disposed within and extending through the tether catheter (202);
a biostimulator (200) including an attachment member (308); and
the attachment device (250) according to any one of claims 1 to 10, wherein the attachment device (250) has a docked configuration in which the attachment device (250) is disposed within the catheter (102) at a first position such that the biostimulator (200) is engaged with the docking cap (122), a tethered configuration in which the attachment device (250) is disposed within the catheter (102) at a second position distal from the first position such that the biostimulator (200) is disengaged with the docking cap (122) and the bulb (530) of the filament (506) remains within the catheter (102), and a release configuration in which the attachment device (250) is disposed within the catheter (102) at a third position distal from the second position such that the bulb (530) of the filament (506) is outside the catheter (102) and removable from the passage (508) of the case (502).

12. The system according to claim 11, wherein the tether (204) includes a bullet (206) at a distal end of the tether (204), the bullet (206) having a diameter larger than a diameter of the tether (204).

13. The system according to claim 12, wherein the bullet (206) has a capsule shape.

14. The system according to any one of claims 11 to 13, wherein the attachment member (308) of the biostimulator (200) defines a hole (310) extending therethrough, the filament (506) passing through the hole (310) to secure the biostimulator (200) to the attachment device (250) when the attachment device (250) is in the docked configuration or the tethered configuration.

15. The system according to claim 14, wherein the filament (506) can be withdrawn from the hole (310) of the attachment member (308) when the attachment device (250) is in the release configuration to decouple the biostimulator (200) from the tether catheter (202).
